# FASCICULE DE BREVET EUROPEEN

(11) **EP 1 699 923 B1**
(45) Date de publication et mention de la délivrance du brevet: **30.07.2008**
(21) Numéro de dépôt: 04817608.5
(22) Date de dépôt: 28.12.2004
(51) Int. Cl.: C12N 15/11

(54) **OLIGONUCLEOTIDE ET SON UTILISATION POUR MODULER L'EXPRESSION DE LA PROTEINKINASE C ISOFORME BETA-1 COMME AGENT DE DEPIGMENTATION CUTANEE**
OLIGONUKLEOTID UND DESSEN VERWENDUNG ZUR MODULATION EINER BETA-1-PROTEINKINASE-ISOFORM C IN FORM EINES HAUTDEPIGMENTIERUNGSMITTELS
OLIGONUCLEOTIDE AND THE USE THEREOF FOR MODULATING AN ISOFORM C BETA-1 PROTEIN-KINASE IN THE FORM OF A SKIN DEPIGMENTATION AGENT

(30) Priorité: 30.12.2003 FR 0315560
(43) Date de publication de la demande: 13.09.2006
(73) Titulaire: L V M H RECHERCHE, 45800 St. Jean de Braye (FR)
(72) Inventeur: KURFURST, Robin, F-45160 Olivet (FR); NIZARD, Carine, F-94200 Ivry sur Seine (FR)
(74) Mandataire: Warcoin, Jacques
(86) Numéro de dépôt international: PCT/FR2004/003397
(87) Numéro de publication internationale: WO 2005/073243

(56) Documents cités:
- WO-A-01/58918
- WO-A-95/02069
- WO-A-97/35998
- PARK H-Y ET AL: "THE BETA ISOFORM OF PROTEIN KINASE C STIMULATES HUMAN MELANOGENESIS BY ACTIVATING TYROSINASE IN PIGMENT CELLS" JOURNAL OF BIOLOGICAL CHEMISTRY, AMERICAN SOCIETY OF BIOLOGICAL CHEMISTS, BALTIMORE, MD, US, vol. 268, no. 16, 5 juin 1993 (1993-06-05), pages 11742-11749, XP002036373 ISSN: 0021-9258
- NISHIZUKA Y: "THE MOLECULAR HETEROGENEITY OF PROTEIN KINASE C AND ITS IMPLICATIONS FOR CELLULAR REGULATION" NATURE, NATURE PUBLISHING GROUP, LONDON, GB, vol. 334, 25 août 1988 (1988-08-25), pages 661-665, XP001118326 ISSN: 0028-0836

## Description

La présente invention concerne des compositions cosmétiques comprenant des séquences oligonucléotidiques ainsi que leurs dérivés capables de s'hybrider avec le gène ou avec les produits du gène codant pour la Protein-Kinase C (PKC) isoforme beta-1(PKC-beta-1).

La présente invention concerne également l'utilisation de ces nouvelles séquences oligonucléotidiques comme agent dépigmentant ou blanchissant de la peau dans une composition cosmétique ou dans une composition dermatologique.

Chez l'homme, la pigmentation résulte de la synthèse et de la distribution des pigments mélaniques dans la peau, les follicules pileux ou les yeux. La pigmentation est génétiquement prédéfinie mais elle est régulée par de nombreux facteurs internes ou externes. Les mélanines produites par les mélanocytes ainsi que le nombre de mélanocytes, leur activité tyrosinasique et leur capacité à exporter les mélanines vers les kératinocytes, la taille des mélanosomes qui contiennent des grains de mélanine, vont conditionner la couleur de la peau humaine. Pour chaque individu, la couleur de la peau varie principalement en fonction de l'irradiation plus ou moins importante des rayons ultra-violets (UV). Autrement dit pour chaque individu, il existe une pigmentation cutanée de base lorsqu'il subit la plus faible irradiation UV, correspondant à sa couleur de peau la plus claire, et une pigmentation cutanée plus intense s'il reçoit une irradiation UV plus forte, allant jusqu'à une pigmentation maximum correspondant à sa couleur de peau la plus foncée lorsqu'il est exposé de manière prolongée à une irradiation UV intense, telle que celle que l'on peut rencontrer en altitude en montagne.

D'autre part, comme on le sait bien, il existe dans la population mondiale, une très grande diversité génétique quant à la pigmentation cutanée. Ainsi, selon les populations, la couleur de la peau correspondant à la pigmentation de base définie ci-dessus, présente une teinte plus ou moins claire se situant entre les deux extrêmes : très claire et très foncée. Egalement selon les populations, la différence de teinte de la peau entre la pigmentation de base et la pigmentation maximum est plus ou moins importante. Ainsi, il est bien connu que les personnes appartenant à certaines populations à peau claire (pigmentation de base) réagissent rapidement et/ou de manière importante à l'action des UV et peuvent donc facilement présenter une peau de teinte foncée, même lorsque ces personnes ne se sont pas exposées volontairement et de façon prolongée au soleil. Dans la suite du texte, ces personnes seront désignées par l'expression « personnes très réactives aux UV ». Il en est notamment ainsi de populations d'origine asiatique ou de certaines populations dites métisses.

Par ailleurs, des personnes voient apparaître sur leur peau, en particulier au niveau du visage ou des mains des zones et/ou des taches plus foncées et/ou plus colorées conférant à la peau une hétérogénéité. Ces taches sont dues à une concentration importante de mélanine dans les kératinocytes de l'épiderme.

Le mécanisme de formation de la pigmentation cutanée fait intervenir la synthèse des mélanines. Ce mécanisme est particulièrement complexe et fait intervenir schématiquement les principales étapes suivantes :
Tyrosine → Dopa → Dopaquinone → Dopachrome → Mélanines

La tyrosinase, activée par une réaction de phosphorylation catalysée par la Protein Kinase C, est une enzyme essentielle intervenant dans cette suite de réactions. La tyrosinase catalyse notamment la réaction de transformation de la tyrosine en Dopa (Dihydroxyphénylalanine) et la réaction de transformation de la Dopa en Dopaquinone conduisant à la formation des pigments mélaniques.

Une molécule est reconnue comme dépigmentante si elle agit directement sur les mélanocytes épidermiques en inhibant l'activité de ces cellules et/ou si elle bloque l'une des étapes de la biosynthèse des mélanines. C'est le cas notamment lorsque la molécule inhibe l'une des enzymes impliquées dans la mélanogénèse
ou lorsqu'elle réagit avec les composés chimiques de la chaîne de synthèse des mélanines.

Les substances dépigmentantes connues sont notamment l'hydroquinone et ses dérivés, l'acide ascorbique et ses dérivés, des extraits placentaires, l'acide kojique, l'arbutine, les iminophénols (WO 99/22707), l'association de carnitine et de quinone (DE 19806947), les dérivés amides d'amino-phénol (FR 2 772 607), et les dérivés de benzothiazole (WO 99/24035). Ces substances peuvent présenter certains inconvénients. Elles peuvent être instables, nécessiter une utilisation à des concentrations élevées, manquer de spécificité quant à leur mode d'action, ou présenter un pouvoir cytotoxique ou irritant.

L'utilisation topique de substances dépigmentantes efficaces et inoffensives est particulièrement recherchée en cosmétique et en dermatologie. On utilise notamment ces substances pour traiter des hyperpigmentations régionales par hyper-activité mélanocytaire telles que les mélasmas idiopathiques, les hyperpigmentations localisées par hyper-activité et prolifération mélanocytaire bénigne telles que les taches pigmentaires dites de sénescence (lentigos séniles), les hyperpigmentations accidentelles telles que la photosensibilisation ou la cicatrisation post-lésionnelle, ainsi que certaines leucodermies telles que le vitiligo. Dans ces derniers cas, à défaut de pouvoir repigmenter la peau, on atténue la pigmentation de la périphérie des zones dépigmentées pour donner à la peau une couleur plus homogène.

Des substances dépigmentantes sont également utilisées en tant qu'agents de blanchiment de la peau par certaines personnes, en particulier celles désignées plus haut, qui sont très réactives aux UV, pour éclaircir leur teint, notamment celui de leur visage et de leurs mains, afin de conserver une couleur de peau la plus claire possible ou tout au moins de réduire les effets pigmentants des rayons UV.

Le problème posé aux professionnels est donc la conception, la fabrication ou l'isolement de nouvelles substances dépigmentantes ou de nouveaux agents blanchissant de la peau humaine, des poils et/ou des cheveux ne présentant pas les inconvénients des substances connues, c'est-à-dire qui soient non irritants, non toxiques et/ou non allergisants pour la peau et stables dans une composition, et qu'ils présentent une activité à très faible concentration sans aucune cytotoxicité.

L'utilisation d'un oligonucléotide antisens pour traiter les maladies dues à un dysfonctionnement des mélanocytes, en particulier le vitiligo et d'autres maladies dépigmentantes, a été décrite dans WO 99/25819. Dans ces pathologies cutanées, l'hypopigmentation résulte d'une teneur anormalement élevée en ténascine. Les oligonucléotides décrits dans ce document agissent contre l'hypopigmentation en régulant l'expression de la ténascine.

Au contraire, l'objet de la présente invention consiste à fournir un agent dépigmentant agissant sur le processus de la mélanogénèse, destiné d'une part, dans le cas d'une pigmentation sensiblement homogène, au blanchiment de la peau, des poils ou des cheveux, c'est-à-dire à diminuer leur pigmentation, et d'autre part, à lutter contre l'hyperpigmentation cutanée à savoir lorsque la peau présente une hétérogénéité de pigmentation.

On connaît de part la demande de brevet WO 01/58918 des oligonucléotides capables de s'hybrider de façon spécifique avec le gène ou un produit du gène codant pour la tyrosinase ou la tyrosinase related-protein 1, enzymes entrant dans le métabolisme de la mélanine. Les séquences décrites permettent de mettre au point des compositions agissant comme agent dépigmentant ou blanchissant de la peau, des poils ou cheveux.

Park et al. (J. of Biol Chem. Vol.268,16, 11742-11749,1993) décrit que l'activation de la tyrosinase par l'isoforme beta de la protéine kinase C (PKC β) stimule la synthèse de la mélanine. Ce document ne décrit pas d'inhibiteurs de la PKC β.

WO 95/02069 décrit des oligonucléotides pour la modulation de l'activité des PKC et notamment de la PKC β1. Cependant, WO 95/02069 ne décrit pas d'applications cosmétiques ou thérapeutiques précises de ces oligonucléotides.

Nishizuka, Y. (Nature, Vol. 334, 25, 661-665) décrit les différents isoformes de la PKC dont les isoformes β1 et β2.

Les inventeurs de la présente invention ont trouvé que, de manière surprenante, des séquences d'oligonucléotide, autres que celles pouvant s'hybrider de façon spécifique avec les enzymes spécifiquement impliquées dans le métabolisme de la mélanine, étaient utiles et efficaces pour agir en tant qu'agent dépigmentant ou blanchissant de la peau, des poils ou cheveux, et ce sans procurer d'effet secondaire.

La présente invention a pour objet une composition cosmétique comprenant un oligonucléotide comportant un nombre de nucléotides compris entre 7 et 25, de préférence égal à 20, capable de s'hybrider de façon spécifique avec les gènes ou les produits des gènes codant pour la protéine kinase C beta-1 (PKC beta-1).

Les inventeurs de la présente invention ont trouvé que des oligonucléotides pouvant s'hybrider de façon spécifique avec le gène ou les produits des gènes (tels que les ARN) codant pour la PKC isoforme beta-1 présentent une activité dépigmentante. Cette activité existe même à très faible concentration, ce qui augmente l'intérêt de ces oligonucléotides. De plus, ces oligonucléotides selon l'invention ne présentent aucune cytotoxicité.

Les oligonucléotides contenus dans la composition cosmétique selon l'invention interviennent en amont des mécanismes de la mélanogénèse en modulant l'expression de la PKC beta-1 et donc son activité. Par conséquent, la diminution de l'activité de la PKC béta-1 conduit à une diminution de la phosphorylation de la tyrosinase dans les mélanocytes.

Ces oligonucléotides offrent une solution idéale aux problèmes posés par les substances utilisées classiquement. Les substances connues qui inhibent l'activité de la tyrosinase (notamment l'hydroquinone et ses dérivés, l'acide ascorbique et ses dérivés, des extraits placentaires, l'acide kojique, l'arbutine) présentent de multiples effets secondaires inacceptables du fait de leur faible spécificité.

La présente invention résout donc les problèmes rencontrés par les recherches antérieures en modulant l'activation de l'enzyme par phosphorylation au lieu d'inhiber directement l'enzyme après son activation pour obtenir l'effet dépigmentant.

Dans la présente invention, le terme "oligonucléotide" signifie des polynucléotides formés à partir de nucléobases naturelles et de groupements pentafuranosyles (sucre) formant des nucléosides qui sont reliés entre eux par liaisons phosphodiesters natives. Le terme "oligonucléotides" se réfère donc aux espèces naturelles ou aux espèces synthétiques formés à partir de sous unités naturelles ou de leurs homologues proches.

Le terme "oligonucléotides" désigne une structure comprenant des nucléotides, de préférence des désoxyribonucléotides, mais également des ribonucléotides. Le terme concerne seulement la structure primaire de la molécule. Ainsi, ce terme inclut l'ADN double et simple brin, aussi bien que l'ARN double et simple brin.

Le terme "oligonucléotides" peut se référer aussi aux parties qui ont des fonctions similaires aux oligonucléotides naturels mais qui peuvent présenter des portions non naturelles. Les oligonucléotides peuvent avoir des parties sucres, des parties nucléobases ou des liaisons internucléotidiques modifiées. Parmi les modifications possibles, les modifications préférées sont les dérivés 2'-O-alkyle sur la partie sucre, en particulier les dérivés 2'-O-éthyloxyméthyle ou 2'-O-méthyle, et/ou les phosphorothioates ou les méthylphosphonates pour le squelette internucléotidique.

Les oligonucléotides chimériques sont compris dans les modifications préférentielles de l'invention. Les oligonucléotides contiennent au moins deux régions chimiquement différentes, chacune comprenant au moins un nucléotide. Il s'agit en particulier d'une ou de plusieurs régions comprenant un nucléotide modifié qui confère une ou plusieurs propriétés bénéfiques comme par exemple une meilleure stabilité biologique, une biodisponiblité accrue, l'augmentation de l'internalisation cellulaire ou l'augmentation de l'affinité pour l'ARN cible.

De façon préférée, le squelette internucléotidique peut être en tout ou partie phosphodiesters, ou phosphorothioates, ou méthylphosphonates ou les combinaisons de liaisons phosphodiesters et/ou phosphorothioates et/ou méthylphosphonates.

Le terme "oligonucléotide" peut également se référer à des oligonucléotides auxquels on a greffé un vecteur d'administration circulaire de type plasmidique ou un vecteur d'administration linéaire de type acide nucléique
ou peptidique.

Dans la présente invention, on entend par :
- "capable de s'hybrider" ou "hybridation", la formation de liaisons hydrogènes, aussi connue comme appariement Watson-Crick entre les bases complémentaires, usuellement sur deux brins d'acide nucléique pour former un duplex en double hélice, ou triplex si l'oligonucléotide est constitué d'un double brin.

Le degré de complémentarité entre deux séquences d'acide nucléique de longueur identique est déterminé en comparant, après alignement, la première séquence avec la séquence complémentaire de la seconde séquence. Le degré de complémentarité est calculé en déterminant le nombre de positions identiques pour lesquelles le nucléotide est identique entre les deux séquences ainsi comparées, en divisant ce nombre de positions identiques par le nombre total de positions et en multipliant le résultat obtenu par 100 pour obtenir le degré de complémentarité entre ces deux séquences.
- "gène codant pour la PKC", la séquence génomique de la PKC comprenant les introns et exons de ce gène.
- "PKC beta-1 ", l'isoforme beta-1 de la PKC
- "produit des gènes codant pour la PKC", les séquences ARN messagers.

L'oligonucléotide contenu dans la composition cosmétique selon l'invention s'hybride de préférence spécifiquement avec le gène ou les produits du gène codant pour la PKC isoforme beta-1. En particulier, l'oligonucléotide de l'invention est capable de s'hybrider avec l'ADN du gène qui code pour la PKC et/ou avec l'ARNm dérivant de ces gènes. Les oligonucléotides selon l'invention comportent un nombre de nucléotides suffisant en identité et en nombre pour s'hybrider de façon spécifique. Cette propriété est couramment appelée "antisens".

Dans la présente invention, le terme "hybridation spécifique" signifie en particulier qu'il existe un degré de complémentarité suffisant pour éviter la fixation non spécifique de l'oligonucléotide sur une séquence non ciblée dans les conditions où la fixation spécifique est souhaitée. Il est entendu que l'oligonucléotide n'a pas besoin de présenter une complémentarité de 100% avec la séquence de l'acide nucléique cible pour s'hybrider spécifiquement. En particulier, un oligonucléotide présentant un degré de complémentarité au moins égal à 80 % environ est capable de s'hybrider spécifiquement avec l'acide nucléique choisi comme cible.

Le rôle d'activateur de la tyrosinase par phosphorylation joué par la PKC et le rôle clé de la tyrosinase dans la mélanogénèse sont connus. L'utilisation d'un oligonucléotide dirigé contre un ARN messager codant pour une enzyme ou une protéine et même la PKC beta-1 afin d'en moduler l'expression est également connue.

Cependant, le rôle de l'isoforme beta-1 de la Protein Kinase C n'était pas connu spécifiquement dans la mélanogénèse. Le caractère ubiquitaire de la PKC fait que la non spécificité d'action des inhibiteurs classiques est rédhibitoire pour un usage dermatologique ou pharmaceutique. De plus, les inhibiteurs classiques de la PKC bêta couvrent les isoformes beta-1 et beta-2 et ne sont donc pas spécifiques des mélanocytes puisque des cellules comme les cellules de Langerhans présentes dans la peau ont une activité PKC beta-2.

La technique élaborée par les inventeurs de la présente invention est la seule qui permette d'avoir une action spécifique sur l'isoforme béta-1 en préservant les autres isoformes de la PKC bêta et de la PKC en général. De plus cette technique n'avait jamais été utilisée comme moyen de dépigmentation.

L'oligonucléotide connu dans la composition cosmétique selon l'invention est déterminé pour s'hybrider directement à l'ARN messager ou au gène. Ils permettent ainsi de réaliser une modulation ultime de la quantité de PKC béta-1 produite par les gènes.

Dans un mode de réalisation préférentiel, la composition cosmétique selon l'invention comprend un oligonucléotide capable de s'hybrider de façon spécifique avec une quelconque des régions 5' à 3', codante ou non codante des gènes codant pour la PKC béta-1.

Dans un mode de réalisation plus préférentiel, la séquence de l'oligonucléotide est l'une des séquences SEQ ID N°1 à SEQ ID N°5 ayant la signification suivante :
- SEQ ID N° 1 : ACACCCCAGGCTCAACGATG
- SED ID N°2 : TGG AGT TTG CAT TCA CCT AC
- SEQ ID N°3 : AAA GGC CTC TAA GAC AAG CT
- SED ID N°4 : GCC AGC ATC TGC ACC GTG AA
- SED ID N°5: CCG AAG CTT ACT CAC AAT TT

Dans un mode de réalisation encore plus préférentiel, la séquence est l'une des séquences SEQ ID N°1 et SEQ ID N°4, et plus particulièrement la séquence SEQ ID N°1.

Dans un autre mode de réalisation préférentiel, l'oligonucléotide comprend une ou plusieurs modifications chimiques au niveau de ses parties sucres, ses parties nucléobases ou son squelette internucléotidique qui confèrent des caractéristiques physico-chimiques améliorées audit oligonucléotide.

Par "caractéristiques physico-chimiques améliorées", on entend des caractéristiques souhaitables de l'oligonucléotide selon l'invention, telles qu'une biodisponibilité accrue, l'augmentation de l'affinité pour les séquences cibles, l'augmentation de l'internalisation cellulaire ou une meilleure stabilité biologique ou l'augmentation de la stabilité en présence de nucléases cellulaires.

A titre d'exemple, les modifications pouvant conférer ces caractéristiques sont les dérivés 2'-O-alkyle et 2'-O-fluoro sur la partie sucre du nucléoside, et les dérivés phosphorothioates ou les dérivés méthylphosphonates au niveau du squelette internucléotidique.

Dans un mode de réalisation préférentiel l'oligonucléotide est modifié chimiquement en ce que :
- une partie des groupements phosphodiesters de son squelette internucléotidique est remplacée par des groupements phosphorothioates.
- une partie des groupements phosphodiesters de son squelette internucléotidique est remplacée par des groupements méthylphosphonates.
- tous les groupements phosphodiesters sont remplacés par des groupements phosphorothioates.
- tous les groupements phosphodiesters sont remplacés par des groupements méthylphosphonates.
- les groupements phosphodiesters sont remplacés en tout ou partie par des groupements phosphorothioates et/ou par des groupements méthylphosphonates.
- on a greffé sur l'oligonucléotide un vecteur d'administration linéaire de type acide nucléique ou peptidique, ou un vecteur d'administration circulaire de type plasmidique.

La présente invention a pour objet une composition cosmétique contenant l'oligonucléotide décrit précédemment et un milieu cosmétiquement acceptable.

Une telle composition peut contenir en outre, un ou plusieurs agents actifs visant à renforcer les effets recherchés.

Lesdits agents actifs utilisables en association avec l'oligonucléotide selon l'invention, utilisés purs ou provenant d'extraits renfermant ces molécules, sont notamment les composés suivants: un oligonucléotide antisens dirigé contre les produits d'expression du gène de la tyrosinase, un oligonucléotide antisens dirigé contre les produits d'expression du gène de la tyrosinase-related-protein 1 (TRP-1), l'acide ellagique et ses dérivés ; l'hydroquinone ; l'arbutine ; le résorcinol et ses dérivés ; la vitamine C et ses dérivés ; le pantothénate sulfonate et ses dérivés ; l'acide kojique ; les extraits placentaires ; des molécules interférant directement ou indirectement avec l'alpha-mélanocyte stimulating hormone (α-MSH) ou son récepteur ou l'hormone adrénocorticotropique (ACTH) ; les polyols tels que la glycérine le glycol ou le propylène glycol ; les vitamines ; les agents kératolytiques et/ou desquamants tels que l'acide salicylique et ses dérivés ; les alpha-hydroxyacides tels que l'acide lactique ou l'acide malique, seuls ou greffés ; l'acide ascorbique et ses dérivés ;les rétinoides et les caroténoides en préparation liposomique ou non tels que le rétinaldéhyde ; le rétinol et ses dérivés tels que le palmitate, le propionate ou l'acétate, le bêta-carotène, des agents antiglycation et/ou antioxydants pris seuls ou en association tels que le tocophérol et ses dérivés, l'ergothionéine, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate; les agents anti-inflammatoires tels que le stéaryl glycyrrhétinate ; les agents apaisants et leurs mélanges, les filtres solaires chimiques ou physiques tels que le méthoxycinnamate d'octyle, le butyl-méthoxydibenzoyl-méthane, l'oxyde de titane et l'oxyde de zinc ; et les acides désoxyribonucléiques et/ou nucléiques.

En cas d'incompatibilité, ces actifs et/ou les oligonucléotides peuvent être incorporés dans des sphérules, notamment des vésicules formés de lipides amphiphiles ioniques ou non ioniques telles que décrites dans le brevet français FR 2534487 et/ou des nanoparticules et/ou des nanospheres.

La composition cosmétique selon l'invention est appropriée pour une utilisation topique et contient donc un milieu cosmétiquement acceptable, c'est-à-dire compatible avec la peau.

La séquence oligonucléotide selon l'invention peut être présente de manière préférentielle en quantité allant de 0,00001% à 10% et de préférence de 0,0003% à 3% du poids total de la composition cosmétique.

La composition de l'invention peut se présenter sous toutes les formes galéniques normalement utilisées pour une application topique notamment sous forme d'une solution aqueuse, hydroalcoolique ou huileuse, d'une émulsion huile dans eau ou eau dans huile ou multiple, d'un gel aqueux ou huileux, d'un produit anhydre liquide, pâteux ou solide, d'une dispersion d'huile dans une phase polymérique telle que les nanosphères et les nanocapsules ou mieux des vésicules lipidiques de type ionique et/ou non-ionique tels que décrit dans le brevet français FR 2534487.

Cette composition peut être plus ou moins fluide et avoir l'aspect d'une crème blanche ou colorée, d'une pommade, d'un lait, d'une lotion, d'un sérum, d'une pâte ou d'une mousse. Elle peut éventuellement être appliquée sur la peau sous forme d'aérosol. Elle peut également se présenter sous forme solide pulvérulent ou non, par exemple sous forme stick. Elle peut encore se présenter sous la forme de patchs, de crayons, de pinceaux et d'applicateurs autorisant une application localisée sur les taches du visage ou des mains. Elle peut être utilisée comme produit de soin et/ou comme produit de maquillage.

De façon connue, la composition de l'invention peut contenir également les adjuvants habituels dans les domaines cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés. Ces adjuvants, selon leur nature, peuvent être introduits dans la phase grasse, dans la phase aqueuse, dans les vésicules lipidiques et/ou dans les nanoparticules.

Dans un mode de réalisation préférentiel selon l'invention, la composition cosmétologique se présente sous la forme d'une émulsion contenant une huile, un émulsionnant choisi parmi les esters d'acide gras et de polyéthylène glycol, tels que le stéarate de PEG-20, et les esters d'acide gras et de glycérine, tels que le stéarate de glycérine, et un co-émulsionnant.

Lorsque la composition cosmétique de l'invention est une émulsion, la proportion de la phase grasse peut aller de 5 à 80% en poids, et de préférence de 5 à 50% en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3% à 30% en poids, et de préférence de 0,5% à 20% en poids par rapport au poids total de la composition.

Comme huiles utilisables en association avec les oligonucléotides selon l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matière grasses des alcools gras (alcool cétylique), des acides gras, des cires (cire de Carnauba, ozokérite).

Comme émulsionnants et coémulsionnants utilisables en association avec les oligonucléotides selon l'invention, on peut citer par exemple les esters d'acide gras et de polyéthylène glycol tels que le stéarate de PEG-20 et les esters d'acide gras et de glycérine tels que le stéarate de glycéryle.

Comme gélifiants hydrophiles utilisables en association avec les oligonucléotides selon l'invention, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles. Comme gélifiants lipophiles, on peut citer les argiles modifiées comme les bentones, les sels métalliques d'acides gras, la silice hydrophobe et les polyéthylènes.

La présente invention a également pour objet l'utilisation d'une séquence oligonucléotide dirigée contre les produits de transcription des gènes codant pour la PKC beta-1 pour la fabrication d'une composition cosmétique.

Cette composition cosmétique est utile pour dépigmenter et/ou blanchir la peau, les poils et/ou les cheveux humains.

La présente invention a également pour objet l'utilisation d'au moins un oligonucléotide à titre d'agent actif inhibiteur de la synthèse de la mélanine pour la fabrication d'une composition pharmaceutique topique destinée au traitement ou à la prévention des hyperpigmentations régionales par hyper-activité mélanocytaire telles que les mélasmas idiopathiques, des hyperpigmentations localisées par hyper-activité et prolifération mélanocytaire bénigne telles que les taches pigmentaires séniles (lentigo actiniques), des hyperpigmentations accidentelles telles que la photosensibilisation ou la cicatrisation post-lésionnelle, et pour le traitement de certaines leucodermies telles que le vitiligo.

Dans un mode de réalisation préférentiel selon l'invention, l'utilisation est caractérisée en ce que le/les oligonucléotides selon l'invention représentent 0,00001 % à 10 %, de préférence 0,0003 % à 3 % du poids total de ladite composition pharmaceutique topique

La composition pharmaceutique est destinée à être administrée de façon simultanée, séparée ou étalée dans le temps en association avec un ou plusieurs actifs.

Les exemples suivants illustrent la présente invention sans toutefois la limiter.

Pour des raisons de stabilité dans les milieux de culture in-vitro et conformément à l'usage, les exemples 2 à 4 ont été réalisés avec des dérivés phosphorothioates et les exemples 5 à 12 ont été réalisés indifféremment avec des dérivés phosphorothioates ou phosphodiesters.

Dans les exemples qui suivent, tous les pourcentages sont donnés en poids, sauf indications contraires.

### Exemple 1 : Synthèse des Oligonucléotides

Les oligonucléotides ont été synthétisés avec un synthétiseur automatique (Perseptive Biosystems Expedite modèle 8909) en utilisant la chimie standard des dérivés phosphoramidites en utilisant les protocoles du constructeur. Les β-cyanoéthyldiisopropylphosphoramidites ont été fournis par la société Perseptive Biosystems. Pour les oligonucléotides phosphodiesters, l'étape d'oxydation du phosphite a été effectuée avec une solution d'iode. En ce qui concerne les oligonucléotides phosphorothioates, l'étape d'oxydation du phosphite a été effectuée en utilisant une solution 0,05 M de 3H-1,2-benzodithiol-3-one 1,1-dioxide dans de l'acétonitrile anhydre. Après clivage de la colonne (Controlled Pore Glass, Perseptive Biosystems) et déprotection totale de la séquence par un traitement de 18h à 55°C par une solution d'ammoniaque à 33%, les oligonucléotides ont été purifiées par précipitation dans l'éthanol en présence d'acétate de sodium. Des contrôles par chromatographie liquide haute pression ont été ensuite réalisés par chromatographie échangeuses d'ions avec élution par un gradient de chlorure de sodium et par chromatographie en phase inverse C18 avec élution par un gradient d'acétonitrile en présence d'acétate de triéthylammonium.

A titre d'exemple, les oligonucléotides suivants ont été synthétisés. Ils sont décrits dans le tableau 1. Il s'agit de cinq séquences de ce tableau, numérotées SEQ ID N°1, SEQ ID N°2, SEQ ID N°3, SEQ ID N°4 et SEQ ID N°5. Leur activité dépigmentante a fait l'objet d'études rapportées dans les exemples suivants, et plus spécifiquement pour la séquence SEQ ID NO.1.

Dans le tableau 1, les numéros mentionnés sous chaque extrémité des séquences indiquent la position de l'oligonucléotide dans les séquences d'origine.

La séquence provient de la séquence dite « HSPB1A» de l'ARN messager codant pour la protéine kinase C type bêta 1 (Genbank accession number X06318).

Par ailleurs, à titre comparatif, et pour confirmer la spécificité des oligonucléotides selon l'invention vis à vis des gènes ou des produits de gènes codant pour la PKC bêta 1, un oligonucléotide basé sur la séquence SEQ ID N°1 de l'invention, a été synthétisé, à savoir, une séquence dite « contrôle sens », désignée SEQ ID N°6 dans le tableau 1, consistant à inverser l'ordre des bases de la séquence SEQ ID N°1.

**TABLEAU 1**

| SEQ ID NO. | SEQUENCE OLIGONUCLEOTIDE | | LOCUS |
|---|---|---|---|
| 1. | ACA CCC CAG GCT CAACGA TG | | HSPKCB1A |
| | 2186 | 2167 | |
| 2. | TGG AGT TTG CAT TCA CCT AC | | HSPKCB1A |
| | 2168 | 2149 | |
| 3. | AAA GGC CTC TAA GAC AAG CT | | HSPKCB1A |
| | 2285 | 2266 | |
| 4. | GCC AGC ATC TGC ACC GTG AA | | HSPKCB1A |
| | 2250 | 2231 | |
| 5. | CCG AAG CTT ACT CAC AAT TT | | HSPKCB1A |
| | 2569 | 2550 | |
| 6. | GTA GCA ACT CGG ACC CCA CA | | HSPKCB1A |
| | 2167 | 2186 | |

### Exemple 2 : Activité anti-PKC bêta 1 de la séquence SEQ ID N°1 sur mélanocytes par western-blot

Les mélanocytes M4Beu sont des cellules isolées de mélanome humain. (R Jacubovich et J.F. Dore Cancer Immunol. Immunother., 7 (1979), 59-64.).

Le milieu de culture utilisé pour ces cellules est le milieu Dubelco's Modified Eagle supplémenté avec 10% de sérum de veau foetal (Gibco, Paisley, GB) et de gentamicine à une concentration de 4µg/ml.

Les cellules M4Beu sont ensemencées à 500 000 cellules par boîte avec la SEQ ID N°1 ou la SEQ ID n°6 à 1µM dans le milieu et pendant 3 jours le milieu est changé une fois avec la SEQ ID N°1 ou la SEQ ID n°6 jusqu'à confluence des cellules, les cellules étant récupérées 24 heures après le dernier traitement.

Lorsque les cellules sont confluentes, le milieu de culture est éliminé et les cellules sont rincées 2 fois avec du PBS. Les cellules sont ensuite collectées par grattage dans 200µl de tampon de lyse complet. La suspension est congelée à -80°C. Le lysat cellulaire est obtenu par sonication à une amplitude de 7µm pendant 2x10s.

Les protéines du lysat cellulaire sont dosées selon la méthode colorimétrique de Bradford en utilisant la micro méthode du kit Biorad (Référence: Bio-Rad protein assay 500-0002, Hercules CA, USA).

L'électrophorèse des protéines est réalisée en minigel de polyacrylamide de 1mm d'épaisseur à 7,5 % en conditions dénaturantes et réductrices, en tampon discontinu selon la méthode de Laemmli (1970). Des gels à 7,5% T, 2,7% C permettent de séparer les protéines d'une masse moléculaire de 30 à 200kDa, ce qui nous permet d'avoir la migration de la PKCβ au milieu du gel.

15 µg de protéine du lysat cellulaire sont déposés, complété avec du tampon de lyse à 15 ou 20µl pour déposer un volume fixe. 4 ou 5 µl respectivement de bleu de migration 4x sont ajoutés aux lysats qui sont ensuite chauffés à 95 % pendant 5mn pour dénaturer les protéines.

L'électrophorèse est réalisée sous réfrigération et à ampérage constant et voltage non limitant.

Après l'électrophorèse, le gel est lavé dans le tampon de transfert, pour une renaturation des protéines. Une membrane de PVDF (polyvinylidènedifuoride,) de bonne tenue mécanique et de forte capacité de fixation des protéines, est également équilibrée dans ce même tampon de transfert.

Le transfert est réalisé par électroélution des protéines hors du gel sur la membrane de PVDF. L'appareil, le Trans-blot SD (semi-dry cell), transfert dans une configuration horizontale. Afin de neutraliser les sites aspécifiques, la membrane est placée dans du lait en poudre demi-écrémé (Régilait^{®}) dissout dans du tampon TBS-T.

Les membranes sont lavées puis incubées avec l'anticorps primaire, c'est-à-dire anti PKC bêta I ou bêta II, sous agitation pendant une heure à température ambiante.

L'anticorps primaire est un anticorps polyclonal de lapin anti PKC bêta I ou bêta II humaine. Il est utilisé à 0,02µg/ml (Santacruz Biotechnology, Santa Cruz, CA, USA). Afin d'éliminer l'excès d'anticorps primaire les membranes sont rincées avec le TBS-T, puis incubées pendant une heure à température ambiante avec l'anticorps secondaire. L'anticorps secondaire est un anti-lapin fait chez le singe couplé à la peroxydase de raifort (Amersham, Buckinghamshire, GB). L'excès d'anticorps est éliminé par rinçages successifs dans le TBS-T.

La détection des protéines se fait par chimioluminescence, utilisant le luminol comme substrat de la peroxydase (kit ECL, Amersham, Buckinghamshire, GB). Après incubation de la membrane avec le luminol et un amplificateur, la membrane est recouverte d'un film autoradiographique (Hyperfilm ECL, Amersham, Buckinghamshire, GB). Le temps d'exposition du film sur la membrane est de 30 minutes. Les taches obtenues sont quantifiées grâce au logiciel "Biolise 3,02V" (BMG LABTECH GmbH, Hanns-Martin-Schleyer-Str. 10, D-77656 Offenburg/Germany). Ce logiciel permet de calculer le volume des taches.

A partir de ces volumes, un pourcentage d'inhibition peut être calculé par rapport à un témoin d'expérience de la manière suivante: [1 - (Vol.de l'échantillon/Vol du témoin)] x100. Les résultats sont présentés dans le Tableau 2 ci-après.

**TABLEAU 2**

| | Pourcentage d'inhibition de la PKC- bêta 1 |
|---|---|
| témoin | 0 |
| SEQ ID N°6 | 5 |
| SEQ ID N°1 | 100 |

### Exemple 3 : Activité anti-PKC beta-1 de la séquence SEQ ID N°1 sur mélanocytes par RT-PCR.

Les mélanocytes M4Beu sont des cellules isolées de mélanome humain. (R Jacubovich et J.F. Dore Cancer Immunol. Immunother., 7 (1979), 59-64.).

Le milieu de culture utilisé pour ces cellules est le milieu Dubelco's Modified Eagle supplémenté avec 10% de sérum de veau foetal (Gibco, Paisley, GB) et de gentamicine à une concentration de 4µg/ml.

Les cellules M4Beu sont ensemencées à 500 000 cellules par boîte avec la SEQ ID N°1 ou la SEQ ID n°6 à 1µM dans le milieu et pendant 3 jours le milieu est changé une fois avec la SEQ ID N°1 ou la SEQ ID n°6 jusqu'à confluence des cellules, les cellules étant récupérées 24 heures après le dernier traitement.

Ensuite, le milieu de culture est éliminé. Le tapis cellulaire est rincé avec du PBS. Les cellules sont incubées 1mn avec une solution de trypsine-EDTA la réaction est stoppée par addition de milieu supplémenté avec du SVF à 10%. La suspension cellulaire obtenue est transférée dans un tube de 15ml et centrifugée pour obtenir le culot cellulaire. Ce culot est ensuite rincé deux fois avec du PBS. Il peut être congelé à sec à -80°C.

C'est à partir de ces culots que l'ARN total sera isolé. Après avoir vérifier que le β-mercaptoéthanol a été ajouté au tampon de lyse SV ARN, 175µl de ce tampon est ajouté au culot cellulaire. La dilution des extraits cellulaires dans une solution de SDS (Sodium Dodecyl Sulfate) contenant une grande concentration en guanidine thiocyanate (tampon de lyse SV ARN) permet de détruire les complexes nucléoprotéiques associés aux ARN et donne ainsi une précipitation sélective des protéines cellulaires, alors que l'ARN reste en solution. Après centrifugation pour nettoyer le lysat des protéines précipitées et des débris cellulaire, l'ARN va être purifié à partir de ce culot. La solution claire du lysat est ainsi récupérée dans un tube propre.

L'ARN est sélectivement précipité par une solution d'éthanol. Cette précipitation est transférée sur la colonne où l'ARN va se lier aux fibres de verre. Après lavage de la colonne avec la solution de lavage SV ARN, les ARN restent fixés sur la colonne.

La Rnase-free Dnase I est appliquée directement sur la colonne pour digérer les ADN génomiques contaminants. La DNase est agitée pendant 15mn, la réaction est stoppée par addition de 200µl de solution Stop SV Dnase sur la colonne.

Puis après lavage avec la solution de lavage SV ARN, les ARN totaux sont finalement élués de la colonne par addition de 100µl d'eau nuclease-free.

Les ARN sont dosés à 260nm. Une unité de densité optique correspond à 40µg /ml d'ARN. Le rapport de l'absorbance à 260nm et à 280nm (DO 260/DO 280) fournit des indications quant à la pureté de l'ARN préparé et doit se situer entre 1,8 et 2 la présence de protéines pouvant diminuer ce rapport.

La concentration (µg/ml) = DO à 260 nm x 40 x facteur de dilution de lecture

La non dégradation de ARNs est vérifiée par électrophorèse d'un aliquot de 2µg en minigel d'agarose à 0,8%. Les ARN sont visualisés grâce au BET.

Le gel est préparé en dissolvant 0,4g d'agarose dans 50ml de tampon tris borate, TBE 1x, par chauffage. Au moment de couler le gel 2,5µl de BET à 10mg/ml sont ajoutés.

La migration s'effectue à 80V pendant 30mn.

Les ARN 18s, 28s et 4s sont colorés au BET et visualisés sous UV (Table Bioblock Scientific, Illkirch, France, longueur d'onde 312nm).

Des échantillons non dégradés laissent apparaître 2 bandes intenses d'ARN 28s et 18s, ainsi qu'une bande moins intense d'ARN 4S.

Pour chaque ARN extrait, deux tubes sont préparés: 1 tube où l'enzyme (Transcription inverse (RT) M-MLV, Gibco, Paisley, GB) sera ajoutée RT+, et 1 où l'enzyme ne sera pas ajoutée RT-. A partir d'un simple brin d'ARN et en présence d'amorces l'enzyme est capable de synthétiser un brin complémentaire.

Les résultats sont présentés dans la Tableau 3.

**TABLEAU 3**

| | RT+ | RT- |
|---|---|---|
| pdN(6) 6U/ml 0,3U ds l'essai | 1 µl | 1 µl |
| ARN total 2µg/µl ds l'essai | 2µg/µl | 2µg/µl |
| H2O | qsp 11,5µl | qsp 11,5µl |

Dans le tableau 3, pdN(6) sont des oligonucléotides composés de 6 nucléotides au hasard, ils serviront d'amorce pour la transcriptase inverse.

Pour dénaturer les ARN, ces tubes sont mis à 65°C pendant 5mn.

Pendant ce temps, le mélange ou mix ci-dessous présenté dans le Tableau 4 est préparé pour chaque tube RT+ et RT-.

**TABLEAU 4**

| MIX | x1 tube |
|---|---|
| Tampon RT 5x 1x dans l'essai | 4µl |
| dNTP (10mM) dans l'essai 500µM | 1µl |
| dTT (0,1 M) dans l'essai 10mM | 2µl |
| RNA guard | 0,5µl |
| TOTAL | 7,5µl |

Dans chaque tube RT+ et RT-, 7,5µl de mix sont ajoutés.

Puis 1 µl d'enzyme RT (200U dans l'essai) est ajoutée dans les tubes RT+, alors que dans les tubes RT-, on ajoute 1 µl d'eau. Puis tous les tubes sont incubés pendant 1 heure à 42°C, température optimum pour que l'enzyme soit la plus efficace. Puis la réaction est stoppée en incubant les tubes à 95°C pendant 5mn.

Ainsi dans les tubes RT+ il y a de l'ADN, la RT ayant synthétisée le brin complémentaire de tous les ARN. Par contre dans les RT- il n'y a pas d'ADN, car il n'y a pas eu d'enzyme. Les tubes RT- servent de contrôle dans la réaction de PCR, pour savoir s'il y a contamination par l'ADN génomique.

Pour chaque tube RT+ et RT-, on réalise une PCR, c'est à dire une amplification enzymatique d'ADN.

2 couples de primer servent d'amorce à l'enzyme (Eurobiotaq® ADN polymerase, Eurobio): couple1 = primer PKCβI/Act1, couple 2 = primer PKCβ/Act2. Dans le même tube, il y aura donc amplification de la PKCβ ou βI et de l'actine. L'actine servant ici de témoin interne.

Pour chaque ADN provenant de la réaction de transcriptase inverse (RT+)
ou non (RT-) les solutions suivantes sont préparées:

| ADN de la RT 100ng dans l'essai | MgCl2 (SOinM) 2mM dans l'essai | H2O | Total |
|---|---|---|---|
| 1µl | 2µl | 12µl | 15µl |

Le mélange suivant présenté dans la Tableau 5 est préparé pour tous les tubes.

Le volume final de réaction est de 50µl.

La réaction se fait dans un appareil de PCR (Crocodile II, Appligène, Illkirch, France)

Les conditions de réaction sont pour le couple 1:
1 cycle de dénaturation des ADN (ouverture des 2 brins) : 5 minutes à 95°C
40 cycles d'amplification des ADN: 30 secondes à 95°C (ouverture des brins)
30 secondes à 56°C (fixation spécifique des amorces)
30 secondes à 72°C (élongation des nouveaux brins)
1 cycle d'élongation des ADN nouvellement formés : 7 minutes à 72°C

**TABLEAU 5**

| Mix PCR | x1 tub |
|---|---|
| Tampon PCR 10x dans l'essai 1x | 5µl |
| dNTP (10mM) dans l'essai 500mM | 1µl |
| Primer actine sens (5µM) dans l'essai 0,05 µM | 0,5µl |
| Primer actine antisens (5µM) dans l'essai 0,05µM | 0,5µl |
| Primer PKCβ ou PKCβI sens (5µM) dans l'essai 1µM | 1µl |
| Primer PKCβ ou PKCβI antisens (5µM) dans l'essai 1µM | 1µl |
| H2O | 25,8µl |
| Taq | 0,2µl |
| Total | 35µl |

Les conditions de réaction sont pour le couple 2 : Ce sont les mêmes cycles de réaction mais la température de fixation spécifique des amorces est de 50°C.

Quantification de l'expression des ARN PKCβ ou βI par rapport à l'expression de l'actine.

Les fragments d'ADN sont déposés sur un gel d'agarose à 2% coloré au BET. La migration électrophorétique se fait à 80V pendant 45mn. Les bandes correspondant aux ARN sont visualisées sous UV (Table UV 312nm, Bioblock scientific , Illkirch, France). 2 bandes apparaissent avec les ADN des tubes RT+ et aucune dans les RT-. Une échelle de poids moléculaires est déposée en même temps pour connaître la taille des bandes obtenues.

Pour le couple 1 le fragment correspondant à l'ARN de la PKCβI est de 547pb alors que le fragment de l'ARN correspondant à l'actine est de 308pb.

Pour le couple 2: le fragment correspondant à l'ARN de la PKCβ est de 3 80pb alors que le fragment de l'ARN correspondant à l'actine est de 514pb.

Les bandes obtenues sont quantifiées grâce au logiciel "Biolise 3,02V". Ce logiciel permet de calculer le volume des bandes.

Nous comparons le rapport du volume des bandes de PKCB/actine ou PKCβI/actine.

Les résultats sont présentés dans le Tableau 6.

**TABLEAU 6**

| | Pourcentage d'expression de l'ARNm codant pour la PKC-bêta 1 |
|---|---|
| SEQ ID N°6 | 0 |
| SEQ ID N°1 | 55 |

### Exemple 4 : Activité anti-tyrosinase de la SEQ ID N°1 sur mélanocytes.

Les mélanocytes M4Beu sont des cellules isolées de mélanome humain. (R Jacubovich et J.F. Dore Cancer Immunol. Immunother., 7 (1979), 59-64.).

Le milieu de culture utilisé pour ces cellules est le Dubelco's Modified Eagle Medium supplémenté avec 10% de sérum de veau foetal (Gibco, Paisley; GB) et de gentamicine à une concentration de 4µg/ml.

Les cellules M4Beu sont ensemencées à 100 000 cellules par boîte avec la SEQ ID N°1 ou la SEQ ID n°6 à 1µM dans le milieu et pendant 3 jours le milieu est changé une fois avec la SEQ ID N°1 ou la SEQ ID n°6 jusqu'à confluence des cellules, les cellules étant récupérées 24 heures après le dernier traitement.

Après 3 lavages des boîtes au sérum physiologique ; avec racloire en plastique, les cellules sont récupérées dans 10 µl de tampon (0,0625 M Tris HCl pH6, SDS 3%, Glycérol 10%). Une l'électrophorèse (Ready gel Tris-glycine 7,5% (Biorad, Hercules, CA, USA, ref 161-09000) tampon de migration Tris-glycine SDS buffer 1X (Biorad, Hercules, CA, USA, ref : 161-0732 ) est réalisée avec un dépôt du lysat cellulaire à la quantité de 30 µg de protéine par puits.

Après migration à 15 mA, le gel est démoulé et rincé 3 fois 20 minutes dans du PBS sous agitation légère pour ramener le pH à 7,5 (pH optimal pour l'activité de la tyrosinase)

La révélation de l'activité tyrosinase est obtenue par incubation du gel 3 heures à 37 °C dans 10 ml d'une solution (1g/l PBS de MBTH Sigma M8006, 1g/l de PBS de DOPA Sigma D9628). le gel est rincé 3 fois au PBS pour arrêter la réaction de la tyrosine sur son substrat. Après photographie, la quantification est ensuite réalisée avec le logiciel "Biolise 3,02V".

Les résultats sont présentés dans la Tableau 7.

**TABLEAU 7**

| | Pourcentage d'inhibition de l'activité de la tyrosinase |
|---|---|
| SEQ ID N°6 | 0 |
| SEQ ID N°1 | 55 |

### Exemple 5 : Poudre pour l'éclaircissement du teint du visage

Une poudre dont la composition est présentée dans le Tableau 8 a été préparée.

**TABLEAU 8**

| | |
|---|---|
| Microcellulose | 20,00% |
| Sodium lauryl sulfoacetate | 15,00% |
| Oligonucléotide SEQ ID NO.1 | 1,00% |
| Parfum, colorants, conservateurs | qs. |
| Talc | Qsp. 100% |

Cette poudre présente une double action. Elle permet un nettoyage de la peau, et de plus elle permet, par un usage régulier durant quelques jours, d'éclaircir le teint. On peut l'appliquer sur la peau du visage une à deux fois par jour.

### Exemple 6 : Emulsion-gel visage de jour dépigmentante

Une émulsion-gel dont la composition est présentée dans le Tableau 8 a été préparée.

**TABLEAU 9**

| | |
|---|---|
| Glycérine | 5,00% |
| triglycérides caprylique/caprique/succinique | 5,00% |
| Méthoxycinnamate d'octyle | 1,00% |
| Diméthicone copolyol | 0,50% |
| Acrylates / C10-30 alkyl acrylate crosspolymer | 0,50% |
| Oligonucléotide SEQ ID N0.4 | 0,01% |
| Neutralisant | qs. |
| Conservateurs, parfum, colorants | qs. |
| Eau | qsp. 100% |

Certaines personnes soumises au rayonnement plus ou moins intense de la lumière du jour, voire du soleil directement, souhaitent conserver un teint clair et éviter l'apparition de taches pigmentées.

L'utilisation de l'émulsion-gel ci-dessus permettra d'atteindre ce but. Cette composition s'applique sur le visage généralement le matin. Elle agit aussi bien de manière préventive que curative sur la pigmentation, régulière ou non, du visage.

### Exemple 7 : Fluide protecteur SPF 30 préventif des taches pigmentaires

Un fluide protecteur dont la composition est présentée dans le Tableau 10 a été préparé.

Le fluide protecteur est utilisé pour prévenir l'apparition de taches pigmentaires, chez les personnes prédisposées à ce phénomène, avant l'exposition à un rayonnement solaire intense. Il est à noter que la présence d'une concentration élevée en filtre solaire permet de compenser la diminution de la protection naturelle, conséquence de la baisse du taux de mélanine.

**TABLEAU 10**

| | |
|---|---|
| Pentacyclométhicone volatile | 49,00% |
| Dioxyde de titane | 15,00% |
| Méthoxycinnamate d'octyle | 7,50% |
| Glycérine | 5,00% |
| Phényltriméthicone | 5,00% |
| Diméthicone copolyol | 3,00% |
| Polyméthylméthacrylate | 2,50% |
| Butyl méthoxydibenzoyle méthane | 1,00% |
| Oligonucléotide SEQ ID NO.1 | 0,1% |
| Neutralisant, Parfum, Conservateurs, antioxydants | qs. |
| Eau | qsp. 100% |

### Exemple 8 : Crème visage dépigmentante

Une crème dont la composition est présentée dans le Tableau 11 a été préparée.

L'utilisation de cette crème permet de traiter les irrégularités de la pigmentation cutanée, en atténuant ou supprimant les taches de sénescence ou les taches pigmentaires actiniques. Elle homogénéise la coloration de la peau et éclaircit le teint.

**TABLEAU 11**

| | |
|---|---|
| Glycéryl stéarate + Peg-100 stéarate | 5,00% |
| Polyisobutène hydrogéné | 4,00% |
| Magnésium ascorbyl phosphate | 3,30% |
| Tricaprylate /caprate de glycérol | 3,00% |
| Squalane | 3,00% |
| Glycérine | 2,00% |
| Cire d'abeille | 1,50% |
| Cétéaryl octanoate | 1,50% |
| Alcool cétylique | 1,00% |
| Alcool stéarylique | 1,00% |
| Diméthicone | 1,00% |
| Gomme xanthane | 0,30% |
| Acide éthylène diamine tétracétique | 0,20% |
| Acide citrique | 0,10% |
| Citrate de sodium | 0,10% |
| Oligonucléotide SEQ ID NO.1 | 0,10% |
| Neutralisant, Parfum, Conservateurs | qs. |
| Eau | qsp. 100% |

### Exemple 9 : Lotion visage pour éclaircir le teint

Une lotion dont la composition est présentée dans le Tableau 12 a été préparée.

**TABLEAU 12**

| | |
|---|---|
| Alcool éthylique | 30,00% |
| PPG-3 Myristyl éther | 5,00% |
| Glycérine | 2,00% |
| Carbomer | 0,20% |
| Polysorbate 20 | 0,20% |
| Oligonucléotide SEQ ID NO. 1 | 0,01% |
| Neutralisant, Parfum, Conservateurs | qs. |
| Eau | qsp. 100% |

Cette lotion pour éclaircir le teint s'utilise après le démaquillage et le nettoyage de la peau.

### Exemple 10 : Sérum Visage éclaircissant

Un sérum dont la composition est présentée dans le Tableau 13 a été préparé.

**TABLEAU 13**

| | |
|---|---|
| Eau | qsp 100% |
| Glycérine | 2% |
| EDTA tetrasodique Acide citrique Citrate trisodique | qsp pH souhaité |
| Gomme xanthane | 0.25% |
| Polyacrylamide, C13.14 isoparaffin, laureth-7 | 0.5% |
| Dimethicone copolyol | 0.25% |
| Oligonucléotide SEQ ID n° 1 | 0.1% |
| Parfum, colorant, conservateur | qs |

Une goutte de cette composition très concentrée de sérum, s'applique sur le visage généralement avant l'application d'une crème pour le visage. Ce sérum s'utilise habituellement par cures d'une à deux semaines pour obtenir ou entretenir un éclaircissement du teint.

### Exemple 11 : Lotion capillaire pour éclaircir la chevelure

Une lotion capillaire dont la composition est présentée dans le Tableau 14 a été préparée.

**TABLEAU 14**

| | |
|---|---|
| Eau | qsp 100% |
| Alcool | 50% |
| Panthenylethyl ether | 0.5% |
| Acétate DL- α -tocophérol | 0.2% |
| Polysorbate 60 | 1% |
| Oligonucléotide SEQ ID NO.1 | 0.01% |
| Parfum | 0.2% |
| Glycerine | 0.5% |
| Colorant | qs |

Cette lotion s'applique sur les cheveux matin et soir pendant la durée nécessaire pour obtenir un éclaircissement progressif de la chevelure. Cette durée est généralement de plusieurs semaines.

### Exemple 12 : Gel crème anti-taches pour les mains

Un gel-crème dont la composition est présentée dans le Tableau 15 a été préparé.

Ce gel-crème doit être appliqué directement sur les taches de sénescence (lentigos séniles) des mains, pour en atténuer la coloration.

**TABLEAU 15**

| | |
|---|---|
| Caprilic/capric diglyceryl succinate | 6% |
| Octyl octanoate | 2.5% |
| Methoxycinnamate d'octyle | 6% |
| Oligonucléotide SEQ ID NO.1 (phosphodiester) | 0.001% |
| Phenyltriméticone | 2.5% |
| Benzophenon-3 | 0.5% |
| Hyaluronate de sodium | 0.05% |
| Gomme xanthane | 0.2% |
| Acrylates/C10.30 alkyl acrylate copolymer | 0.5% |
| Glycérine | 2% |
| PEG 150 | 3% |
| Neutralisants, Colorants, parfum, conservateurs | qs |
| Eau purifiée | qsp 100% |

### Exemple 13 : Solution dermatologique pour traiter l'hyperpigmentation d'origine pathologique.

Un sérum dont la composition est présentée dans le tableau 16 a été préparé.

**TABLEAU 16**

| | |
|---|---|
| Pentacyclométhicone volatile | 49,00% |
| Dioxyde de titane | 15,00% |
| Méthoxycinnamate d'octyle | 7,50% |
| Glycérine | 5,00% |
| Phényltriméthicone | 5,00% |
| Diméthicone copolyol | 3,00% |
| Polyméthylméthacrylate | 2,50% |
| Butyl méthoxydibenzoyle méthane | 1,00% |
| Oligonucléotide SEQ ID NO.1 | 2,00% |
| Neutralisant, Parfum, Conservateurs, antioxydants | qs. |
| Eau | qsp. 100% |

Ce sérum s'applique sur la peau quotidiennement pour traiter les personnes atteintes d'hyperpigmentations régionales.

## Revendications

1. Composition cosmétique topique comprenant au moins un oligonucléotide comportant un nombre de nucléotides compris entre 7 et 25, de préférence égal à 20, capable de s'hybrider de façon spécifique avec les gènes ou les produits des gènes codant pour la protéine kinase C béta-1 (PKC béta-1).

2. Composition cosmétique topique selon la revendication 1, comprenant au moins un oligonucléotide capable de s'hybrider de façon spécifique avec une quelconque des régions 5' à 3', codante ou non codante des gènes codant pour la PKC béta-1

3. Composition cosmétique topique selon l'une des revendications 1 ou 2 comprenant au moins un oligonucléotide dont la séquence est l'une des séquences SEQ ID N°1 à SEQ ID N°5 ayant la signification suivante :
- SEQ ID N° 1 : ACACCCCAGGCTCAACGATG
- SEQ ID N° 2 : TGGAGTTTGCATTCACCTAC
- SED ID N° 3 : AAA GGC CTC TAA GAC AAG CT
- SED ID N° 4 : GCC AGC ATC TGC ACC GTG AA
- SED ID N° 5 : CCGAAGCTTACTCACAATTT

4. Composition cosmétique topique selon la revendication 3, comprenant au moins un oligonucléotide dont la séquence est l'une des séquences SEQ ID N°1 et SEQ ID N° 4.

5. Composition cosmétique topique selon la revendication 3, comprenant au moins un oligonucléotide dont la séquence est SEQ ID N°1.

6. Composition cosmétique topique selon l'une des revendications précédentes, comprenant au moins un oligonucléotide comprenant une ou plusieurs modifications chimiques au niveau de ses parties sucres, ses parties nucléobases ou son squelette internucléotidique, lesdites modifications conférant des caractéristiques physico-chimiques améliorées audit oligonucléotide.

7. Composition cosmétique topique selon la revendication 6, comprenant au moins un oligonucléotide dont la partie sucre comprend un substituant 2'-0-fluoro ou 2'-O-alkyle, préférentiellement un substituant 2'-O-éthyloxyméthyle ou 2'-O-méthyle.

8. Composition cosmétique topique selon la revendication 6 ou 7, comprenant au moins un oligonucléotide dont une partie des groupements phosphodiesters de son squelette internucléotidique est remplacée par des groupements phosphorothioates.

9. Composition cosmétique topique selon la revendication 6 ou 7, comprenant au moins un oligonucléotide dont une partie des groupements phosphodiesters de son squelette internucléotidique est remplacée par des groupements méthylphosphonates.

10. Composition cosmétique topique selon la revendication 6 ou 7, comprenant au moins un oligonucléotide dont tous les groupements phosphodiesters sont remplacés par des groupements phosphorothioates.

11. Composition cosmétique topique selon la revendication 6 ou 7, comprenant au moins un oligonucléotide dont tous les groupements phosphodiesters sont remplacés par des groupements méthylphosphonates.

12. Composition cosmétique topique selon les revendications 6 ou 7, comprenant au moins un oligonucléotide dont les groupements phosphodiesters sont remplacés en tout ou partie par des groupements phosphorothioates et/ou par des groupements méthylphosphonates.

13. Composition cosmétique topique selon l'une des revendications 1 à 12, comprenant au moins un oligonucléotide auquel on a greffé un vecteur d'administration linéaire de type acide nucléique ou peptidique, ou un vecteur d'administration circulaire de type plasmidique.

14. Composition cosmétique topique selon l'une quelconque des revendications 1 à 13 contenant un ou plusieurs agents actifs choisis parmi un oligonucléotide antisens dirigé contre les produits d'expression du gène de la tyrosinase ; un oligonucléotide antisens dirigé contre les produits d'expression du gène de la tyrosinase-related-protein 1 (TRP-1) ; l'acide ellagique et ses dérivés ; le résorcinol et ses dérivés ; la vitamine C et ses dérivés ; le pantothénate sulfonate et ses dérivés ; des molécules interférant directement ou indirectement avec l'alpha-mélanocyte stimulating hormone (α-MSH) ou son récepteur ou l'hormone adrénocorticotropique (ACTH) ; les polyols tels que la glycérine, le glycol ou le propylène glycol ; les vitamines ; les agents kératolytiques et/ou desquamants tels que l'acide salicylique et ses dérivés ; les alpha-hydroxyacides tels que l'acide lactique ou l'acide malique, seuls ou greffés ; l'acide ascorbique et ses dérivés ; les rétinoïdes et les caroténoïdes en préparation liposomique ou non, tels que le rétinaldéhyde ; le rétinol et ses dérivés tels que le palmitate le propionate ou l'acétate, le béta-carotène; des agents antiglycations et/ou antioxydants pris seuls ou en association tels que le tocophérol et ses dérivés, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate ; les agents anti-inflammatoires tels que le stéaryl glycyrrhétinate ; les agents apaisants et leurs mélanges, les filtres solaires chimiques ou physiques tels que le méthoxycinnamate d'octyle, le butyl-méthoxydibenzoyl-méthane, l'oxyde de titane et l'oxyde de zinc.

15. Composition cosmétique topique selon l'une des revendications 1 à 14, **caractérisée en ce que** le/les oligonucléotides selon l'invention représentent 0,00001 % à 10 %, de préférence 0,0003 % à 3 % du poids total de la composition.

16. Composition cosmétique selon l'une quelconque des revendications 1 à 15 se présentant sous la forme d'une émulsion contenant une huile, un émulsionnant choisi parmi les esters d'acide gras et de polyéthylène glycol, tels que le stéarate de PEG-20, et les esters d'acide gras et de glycérine, tels que le stéarate de glycérine, et un co-émulsionnant.

17. Utilisation d'une composition cosmétique selon l'une quelconque des revendications 1 à 16 pour dépigmenter ou blanchir la peau, les poils et/ou les cheveux humains.

18. Utilisation d'un oligonucléotide comportant un nombre de nucléotides compris entre 7 et 25, de préférence égal à 20, capable de s'hybrider de façon spécifique avec les gènes ou les produits des gènes codant pour la protéine kinase C béta-1 (PKC béta-1) pour l'obtention d'un médicament destiné au traitement ou à la prévention des hyperpigmentations régionales par hyper-activité mélanocytaire telles que les mélasmas idiopathiques, des hyperpigmentations localisées par hyper-activité et prolifération mélanocytaire bénigne telles que les taches pigmentaires séniles (lentigo actiniques), des hyperpigmentations accidentelles telles que la photosensibilisation ou la cicatrisation post-lésionnelle, et pour le traitement de certaines leucodermies telles que le vitiligo.

19. Utilisation selon la revendication 18 dans laquelle l'oligonucléotide est capable de s'hybrider de façon spécifique avec une quelconque des régions 5' à 3', codante ou non codante des gènes codant pour la PKC béta-1

20. Utilisation selon l'une des revendications 18-19 dans laquelle l'oligonucléotide a l'une des séquences SEQ ID N° 1 à SEQ ID N°5 ayant la signification suivante
- SEQ ID N° 1 : ACACCCCAGGCTCAACGATG
- SED ID N° 2 : TGGAGTTTGCATTCACCTAC
- SEQ ID N° 3 : AAA GGC CTC TAA GAC AAG CT
- SED ID N° 4 : GCCAGCATCTGCACCGTGAA
- SED ID N° 5 : CCG AAG CTT ACT CAC AAT TT

21. Utilisation selon l'une des revendications 18-20 dans laquelle l'oligonucléotide a l'une des séquences SEQ ID N° 1 et SEQ ID N° 4.

22. Utilisation selon l'une des revendications 18-21 dans laquelle l'oligonucléotide a la séquence SEQ ID N° 1.

23. Utilisation selon l'une des revendications 18-22 dans laquelle l'oligonucléotide comprend une ou plusieurs modifications chimiques au niveau de ses parties sucres, ses parties nucléobases ou son squelette internucléotidique, lesdites modifications conférant des caractéristiques physico-chimiques améliorées audit oligonucléotide.

24. Utilisation selon la revendication 23 dans laquelle la partie sucre de l'oligonucléotide comprend un substituant 2'-O-fluoro ou 2'-O-alkyle, préférentiellement un substituant 2'-, O-éthyloxyméthyle ou 2'-O-méthyle.

25. Utilisation selon l'une des revendications 23-24 dans laquelle une partie des groupements phosphodiesters du squelette internucléotidique de l'oligonucléotide est remplacée par des groupements phosphorothioates.

26. Utilisation selon l'une des revendications 23-24 dans laquelle une partie des groupements phosphodiesters du squelette internucléotidique de l'oligonucléotide est remplacée par des groupements méthylphosphonates.

27. Utilisation selon l'une des revendications 23-24 dans laquelle tous les groupements phosphodiesters de l'oligonucléotide sont remplacés par des groupements phosphorothioates.

28. Utilisation selon l'une des revendications 23-24 dans laquelle tous les groupements phosphodiesters de l'oligonucléotide sont remplacés par des groupements méthylphosphonates.

29. Utilisation selon l'une des revendications 23-24 dans laquelle les groupements phosphodiesters de l'oligonucléotide sont remplacés en tout ou partie par des groupements phosphorothioates et/ou par des groupements méthylphosphonates.

30. Utilisation selon l'une des revendications 18-29 dans laquelle on a greffé un vecteur d'administration linéaire de type acide nucléique ou peptidique, ou un vecteur d'administration circulaire de type plasmidique à l'oligonucléotide.

## Claims

1. A topical cosmetic composition comprising at least one oligonucleotide including a number of nucleotides comprised between 7 and 25, preferably equal to 20, capable of specifically hybridizing with the genes or the products of the genes encoding protein kinase C beta-1 (PKC beta-1).

2. The topical cosmetic composition according to claim 1, comprising at least one oligonucleotide capable of specifically hybridizing with any of the coding or non-coding 5' to 3' regions of the genes coding for PKC beta-1.

3. The topical cosmetic composition according to any of claims 1 or 2 comprising at least one oligonucleotide, the sequence of which is one of the sequences SEQ ID No.1 to SEQ ID No.5 with the following meaning:
- SEQ ID No.1: ACACCCCAGGCTCAACGATG
- SEQ ID No.2: TGGAGTTTGCATTCACCTAC
- SEQ ID No.3: AAA GGC CTC TAA GAC AAG CT
- SEQ ID No.4: GCC AGC ATC TGC ACC GTG AA
- SEQ ID No.5: CCGAAGCTTACTCACAATTT.

4. The topical cosmetic composition according to claim 3, comprising at least one oligonucleotide, the sequence of which is one of the sequences SEQ ID No. 1 and SEQ ID No.4.

5. The topical cosmetic composition according to claim 3, comprising at least one oligonucleotide, the sequence of which is SEQ ID No.1.

6. The topical cosmetic composition according to any of the preceding claims, comprising at least one oligonucleotide comprising one or more chemical modifications in its sugar portions, its nucleobase portions or its internucleotide backbone, said modifications imparting enhanced physico-chemical characteristics to said oligonucleotide.

7. The topical cosmetic composition according to claim 6, comprising at least one oligonucleotide, the sugar portion of which comprises a 2'-O-fluroro or 2'-O-alkyl substituent, preferentially a 2'-O-ethyloxymethyl or 2'-O-methyl substituent.

8. The topical cosmetic composition according to claim 6 or 7, comprising at least one oligonucleotide, a portion of the phosphodiester groups of its internucleotide backbone being replaced with phosphorothioate groups.

9. The topical cosmetic composition according to claim 6 or 7, comprising at least one oligonucleotide, a portion of the phosphodiester groups of its internucleotide backbone being replaced with methylphosphonate groups.

10. The topical cosmetic composition according to claim 6 or 7, comprising at least one oligonucleotide, all its phosphodiester groups being replaced with phosphorothioate groups.

11. The topical cosmetic composition according to claim 6 or 7, comprising at least one oligonucleotide, all its phosphodiester groups being replaced with methylphosphonate groups.

12. The topical cosmetic composition according to claim 6 or 7, comprising at least one oligonucleotide, its phosphodiester groups being totally or partly replaced with phosphorothioate groups and/or methylphosphonate groups.

13. The topical cosmetic composition according to any of claims 1 to 12, comprising at least one oligonucleotide to which a linear administration vector of the nucleic acid or peptide type or a circular administration vector of the plasmid type is grafted.

14. The topical cosmetic composition according to any of claims 1 to 13, containing one or more active agents selected from an antisense oligonucleotide directed against the products of expression of the gene of tyrosinase; an antisense oligonucleotide directed against the products of expression of the gene of tyrosinase-related protein-1 (TRP-1); ellagic acid and its derivatives; resorcinol and its derivatives; vitamin C and its derivatives; pantothenate sulfonate and its derivatives; molecules directly or indirectly interfering with alpha-melanocyte stimulating hormone (α-MSH) or its receptor or adrenocorticotropic hormone (ACTH); polyols such as glycerine, glycol or propylene glycol; vitamins; keratolytic and/or desquamating agents such as salicylic acid and its derivatives; alpha-hydroxy acids such as lactic acid or malic acid, either alone or grafted; ascorbic acid and its derivatives; retinoids and carotenoids either in a liposome preparation or not, such as retinaldehyde; retinol and its derivatives such as palmitate, propionate or acetate, beta-carotene; anti-glycation and/or antioxidant agents taken alone or in association such as tocopherol and its derivatives, thiotaurine, hypotaurine, aminoguanidine, thiamine pyrophosphate, pyridoxamine, lysine, histidine, arginine, phenylalanine, pyridoxine, adenosine triphosphate; anti-inflammatory agents such as stearyl glycyrrhetinate; soothing agents and their mixtures, chemical or physical solar filters such as octyl methoxycinnamate, butyl-methoxydibenzoylmethane, titanium oxide and zinc oxide.

15. The topical cosmetic composition according to any of claims 1 to 14, **characterized in that** the oligonucleotide(s) according to the invention represent(s) 0.00001% to 10%, preferably 0.0003% to 3% of the total weight of the composition.

16. The cosmetic composition according to any of claims 1 to 15 appearing as an emulsion containing an oil, an emulsifier selected from fatty acid and polyethylene glycol esters, such as PEG-20 stearate, and fatty acid and glycerine esters, such as glycerine stearate, and a co-emulsifier.

17. The use of a cosmetic composition according to any of claims 1 to 16 for depigmenting or whitening skin, body hairs and/or human head hair.

18. The use of an oligonucleotide including a number of nucleotides comprised between 7 and 25, preferably equal to 20, capable of specifically hybridizing with the genes or the products of genes encoding protein kinase C beta-1 (PKC beta-1) in order to obtain a drug intended for treating or preventing regional hyperpigmentations by melanocyte hyperactivity such as idiopathic melasmas, localized hyperpigmentations by hyperactivity and benign melanocyte proliferation such as senile pigmentary spots (actinic lentigos), accidental hyperpigmentations such as photosensitization or post-lesional scarring, and for the treatment of certain leukodermas such as vitiligo.

19. The use according to claim 18, wherein the oligonucleotide is capable of specifically hybridizing with any of the coding or non-coding 5' to 3' regions of genes coding for PKC beta-1.

20. The use according to any of claims 18-19 wherein the oligonucleotide has one of the sequences SEQ ID No.1 to SEQ ID No.5 having the following meaning
- SEQ ID No.1: ACACCCCAGGCTCAACGATG
- SEQ ID No.2: TGGAGTTTGCATTCACCTAC
- SEQ ID No.3: AAA GGC CTC TAA GAC AAG CT
- SEQ ID No.4: GCCAGCATCTGCACCGTGAA
- SEQ ID No.5: CCG AAG CTT ACT CAC AAT TT.

21. The use according to any of claims 18-20 wherein the oligonucleotide has one of the sequences SEQ ID No.1 and SEQ ID No.4.

22. The use according to any of claims 18-21 wherein the oligonucleotide has the sequence SEQ ID No.1.

23. The use according to any of claims 18-22 wherein the oligonucleotide comprises one or more chemical modifications in its sugar portions, its nucleobase portions, or its internucleotide backbone, said modifications imparting enhanced physico-chemical characteristics to said oligonucleotide.

24. The use according to claim 23 wherein the sugar portion of the oligonucleotide comprises a 2'-O-fluoro or 2' -O-alkyl substituent, preferably a 2'-O-ethoxymethyl or 2'-O-methyl substituent.

25. The use according to any of claims 23-24, wherein a portion of the phosphodiester groups of the oligonucleotide internucleotide backbone is replaced with phosphorothioate groups.

26. The use according to any of claims 23-24 wherein a portion of the phosphodiester groups of the oligonucleotide internucleotide backbone is replaced with methylphosphonate groups.

27. The use according to any of claims 23-24 wherein all the phosphodiester groups of the oligonucleotide are replaced with phosphorothiate groups.

28. The use according to any of claims 23-24 wherein all the phosphodiester groups of the oligonucleotide are replaced with methylphosphonate groups.

29. The use according to any of claims 23-24 wherein the phosphodiester groups of the oligonucleotide are totally or partly replaced with phosphorothioate groups and/or methylphosphonate groups.

30. The use according to any of claims 18-29 wherein a linear administration vector of the nucleic acid or peptide type or a circular administration vector of the plasmid type, was grafted to the oligonucleotide.

## Patentansprüche

1. Topische kosmetische Zusammensetzung, mindestens ein Oligonukleotid umfassend, das eine Nukleotidanzahl zwischen 7 und 25 inklusive aufweist, vorzugsweise von gleich 20, das in der Lage ist, auf spezifische Weise mit den Genen oder den Produkten der Gene zu hybridisieren, die für die Beta-1-Proteinkinase-Isoform C (PKC-Beta 1) codieren.

2. Topische kosmetische Zusammensetzung nach Anspruch 1, mindestens ein Oligonukleotid umfassend, das in der Lage ist, auf spezifische Weise mit einer der Regionen 5' bis 3' zu hybridisieren, die für die Gene codiert oder nicht codiert, die für die PKC-Beta 1 codieren.

3. Topische kosmetische Zusammensetzung nach einem der Ansprüche 1 oder 2, mindestens ein Oligonukleotid umfassend, dessen Sequenz eine der Sequenzen SEQ ID Nr. 1 bis SEQ ID Nr. 5 mit der folgenden Bedeutung ist:
- SEQ ID Nr. 1: ACACCCCAGGCTCAACGATG
- SEQ ID Nr. 2: TGGAGTTTGCATTCACCTAC
- SEQ ID Nr. 3: AAA GGC CTC TAA GAC AAG CT
- SEQ ID Nr. 4: GCC AGC ATC TGC ACC GTG AA
- SEQ ID Nr. 5: CCGAAGCTTACTCACAATTT

4. Topische kosmetische Zusammensetzung nach Anspruch 3, mindestens ein Oligonukleotid umfassend, dessen Sequenz eine der Sequenzen SEQ ID Nr. 1 und SEQ ID Nr. 4 ist.

5. Topische kosmetische Zusammensetzung nach Anspruch 3, mindestens ein Oligonukleotid umfassend, dessen Sequenz SEQ ID Nr. 1 ist.

6. Topische kosmetische Zusammensetzung nach einem der vorangehenden Ansprüche, mindestens ein Oligonukleotid umfassend, das eine oder mehrere chemische Modifizierungen auf Ebene seiner Zuckerabschnitte, seiner Nukleobasenabschnitte oder seines Internukleotidskelett aufweist, wobei die Modifizierungen dem Oligonukleotid verbesserte physikalisch-chemische Eigenschaften verleihen.

7. Topische kosmetische Zusammensetzung nach Anspruch 6, mindestens ein Oligonukleotid umfassend, dessen Zuckerabschnitt einen 2'-O-Fluor- oder 2'-O-Alkylsubstituenten aufweist, vorzugsweise einen 2'-O-Ethyloxymethyl- oder 2'-O-Methylsubstituenten.

8. Topische kosmetische Zusammensetzung nach Anspruch 6 oder 7, mindestens ein Oligonukleotid umfassend, von dem ein Abschnitt der Phosphordiestergruppen seines Internukleotidskeletts durch Phosphorthioatgruppen ersetzt ist.

9. Topische kosmetische Zusammensetzung nach Anspruch 6 oder 7, mindestens ein Oligonukleotid umfassend, von dem ein Abschnitt der Phosphordiestergruppen seines Internukleotidskeletts durch Methylphosphonatgruppen ersetzt ist.

10. Topische kosmetische Zusammensetzung nach Anspruch 6 oder 7, mindestens ein Oligonukleotid umfassend, bei dem alle Phosphordiestergruppen durch Phosphorthioatgruppen ersetzt sind.

11. Topische kosmetische Zusammensetzung nach Anspruch 6 oder 7, mindestens ein Oligonukleotid umfassend, bei dem alle Phosphordiestergruppen durch Methylphosphonatgruppen ersetzt sind.

12. Topische kosmetische Zusammensetzung nach den Ansprüchen 6 oder 7, mindestens ein Oligonukleotid umfassend, dessen Phosphordiestergruppen ganz oder teilweise durch Phosphorthioatgruppen und/oder Methylphosphonatgruppen ersetzt sind.

13. Topische kosmetische Zusammensetzung nach einem der Ansprüche 1 bis 12, mindestens ein Oligonukleotid umfassend, auf das ein Vektor zur linearen Applikation vom Typ Nuklein- oder Peptidsäure gepfropft wurde, oder ein Vektor zur zirkularen Applikation vom Typ Plasmid.

14. Topische kosmetische Zusammensetzung nach einem der Ansprüche 1 bis 13, einen oder mehrere aktive Wirkstoffe umfassend, die ausgewählt sind aus einem Antisense-Oligonukleotid, das gegen die Expressionsprodukte des Gens der Tyrosinase gerichtet ist; einem Antisense-Oligonukleotid, das gegen die Expressionsprodukte des Gens von Tyrosinase related protein 1 (TRP-1) gerichtet ist; der Ellaginsäure und ihren Derivaten; dem Resorcin und seinen Derivaten; dem Vitamin C und seinen Derivaten; dem Pantothenatsulfonat und seinen Derivaten; den Molekülen, die direkt oder indirekt mit dem Alpha-Melanocyte stimulating hormone (α-MSH) oder seinem Rezeptor oder dem Adrenocorticotropin-Hormon (ACTH) interferieren; den Polyolen wie Glycerin, Glycol oder Propylenglycol; den Vitaminen; den keratolytischen und/oder abschuppenden Wirkstoffen wie die Salicylsäure und ihre Derivate; den Alpha-Hydroxysäuren wie die Milchsäure oder die Apfelsäure, allein oder gepfropft; der Ascorbinsäure und ihren Derivaten; den Retinoiden und den Karotinoiden in liposomischer Zubereitung oder nicht, wie das Retinaldehyd; dem Retinol und seinen Derivaten wie das Palmitat, das Propionat oder das Acetat, das Beta-Karotin; den Anti-Glykations- und/oder antioxidativen Wirkstoffen, allein oder in Kombination mit solchen wie dem Tocopherol und seinen Derivaten, dem Thiotaurin, dem Hypotaurin, dem Aminoguanidin, dem Thiamin-Pyrophosphat, dem Pyridoxamin, dem Lysin, dem Histidin, dem Arginin, dem Phenylalanin, dem Pyridoxin, dem Adenosintriphosphat herangezogen; den entzündungshemmenden Wirkstoffen wie das Stearylglycyrrhetinat; den besänftigenden Wirkstoffen und ihren Gemischen, den chemischen oder physikalischen Sonnenfiltern wie das Octylmethoxycinnamat, das Butylmethoxydibenzoylmethan, das Titanoxid und das Zinkoxid.

15. Topische kosmetische Zusammensetzung nach einem der Ansprüche 1 bis 14, **dadurch gekennzeichnet, dass** das oder die erfindungsgemäßen Oligonukleotide 0,00001 % bis 10 %, vorzugsweise 0,0003 % bis 3 % des Gesamtgewichts der Zusammensetzung darstellen.

16. Topische kosmetische Zusammensetzung nach einem der Ansprüche 1 bis 15, die sich in Form einer Emulsion darstellt, die ein Öl enthält, einen Emulgator, der aus den Fettsäure- und Polyethylenglykolestern ausgewählt ist, wie das PEG-20-Stearat, und den Fettsäure- und Glycerinestern, wie das Glycerinstearat, und einen Co-Emulgator.

17. Verwendung einer kosmetischen Zusammensetzung nach einem der Ansprüche 1 bis 16, um die Haut, die Körperbehaarung und/oder das Haar des Menschen zu depigmentieren oder zu bleichen.

18. Verwendung eines Oligonukleotids, das eine Nukleotidanzahl zwischen 7 und 25 inklusive umfasst, vorzugsweise von gleich 20, das imstande ist, auf spezifische Weise mit den Genen oder den Produkten der Gene zu hybridisieren, die für die Beta-1-Proteinkinase C (PKC-Beta 1) codieren zur Herstellung eines Arzneimittels, das zur Behandlung oder Vorbeugung regionaler Hyperpigmentationen durch Hyperaktivität der Melanozyten wie idiopathisches Melasma bestimmt ist, lokaler Hyperpigmentationen durch Hyperaktivität und gutartige Ausbreitung der Melanozyten, wie altersbedingte Pigmentflecke (Altersflecke), zufälliger Hyperpigmentationen wie die Photosensibilisierung oder die Narbenbildung nach Verletzungen, und zur Behandlung bestimmter Leucodermien wie Vitiligo.

19. Verwendung nach Anspruch 18, wobei das Oligonukleotid in der Lage ist, auf spezifische Weise mit einer der Regionen 5' bis 3' zu hybridisieren, die für die Gene codiert oder nicht codiert, die für die PKC-Beta 1 codieren.

20. Verwendung nach einem der Ansprüche 18 bis 19, wobei das Oligonukleotid eine der Sequenzen SEQ ID Nr. 1 bis SEQ ID Nr. 5 mit der folgenden Bedeutung hat:
- SEQ ID Nr. 1: ACACCCCAGGCTCAACGATG
- SEQ ID Nr. 2: TGGAGTTTGCATTCACCTAC
- SEQ ID Nr. 3: AAA GGC CTC TAA GAC AAG CT
- SEQ ID Nr. 4: GCC AGC ATC TGC ACC GTG AA
- SEQ ID Nr. 5: CCG AAG CTT ACT CAC AAT TT

21. Verwendung nach einem der Ansprüche 18 bis 20, wobei das Oligonukleotid eine der Sequenzen SEQ ID Nr. 1 und SEQ ID Nr. 4 hat.

22. Verwendung nach einem der Ansprüche 18 bis 21, wobei das Oligonukleotid die Sequenz SEQ ID Nr. 1 hat.

23. Verwendung nach einem der Ansprüche 18 bis 22, wobei das Oligonukleotid eine oder mehrere chemische Modifizierungen auf Ebene seiner Zuckerabschnitte, seiner Nukleobasenabschnitte oder seines Internukleotidskelett aufweist, wobei die Modifizierungen dem Oligonukleotid verbesserte physikalisch-chemische Eigenschaften verleihen.

24. Verwendung nach Anspruch 23, wobei der Zuckerabschnitt des Oligonukleotids einen 2'-O-Fluor-oder 2'-O-Alkylsubstituenten, vorzugsweise einen 2'-O-Ethyloxymethyl- oder 2'-O-Methylsubstituenten, aufweist.

25. Verwendung nach einem der Ansprüche 23 bis 24, wobei ein Abschnitt der Phosphordiestergruppen des Internukleotidskeletts des Oligonukleotids durch Phosphorthioatgruppen ersetzt ist.

26. Verwendung nach einem der Ansprüche 23 bis 24, wobei ein Abschnitt der Phosphordiestergruppen des Internukleotidskeletts des Oligonukleotids durch Methylphosphonatgruppen ersetzt ist.

27. Verwendung nach einem der Ansprüche 23 bis 24, wobei alle Phosphordiestergruppen des Oligonukleotids durch Phosphorthioatgruppen ersetzt sind.

28. Verwendung nach einem der Ansprüche 23 bis 24, wobei alle Phosphordiestergruppen des Oligonukleotids durch Methylphosphonatgruppen ersetzt sind.

29. Verwendung nach einem der Ansprüche 23 bis 24, wobei die Phosphordiestergruppen des Oligonukleotids ganz oder teilweise durch Phosphorthioatgruppen und/oder Methylphosphonatgruppen ersetzt sind.

30. Verwendung nach einem der Ansprüche 18 bis 29, wobei ein Vektor zur linearen Applikation vom Typ Nuklein- oder Peptidsäure oder ein Vektor zur zirkularen Applikation vom Typ Plasmid auf das Oligonukleotid gepfropft wurde.


### LISTE DE SEQUENCES

<110> LOUIS VUITTON MOËT HENNESSY (LVM)
<120> Oligonucléotides anti-PKC
<130> D21706
<160> 5
<170> Patentin version 3.2
<210> 1
   <211> 20
   <212> ADN
   <213> Séquence artificielle
<220>
   <223> Oligonucléotide
<400> 1
   acaccccagg ctcaacgatg 20
<210> 2
   <211> 20
   <212> ADN
   <213> Séquence artificielle
<220>
   <223> Oligonucléotide
<400> 2
   tggagtttgc attcacctac 20
<210> 3
   <211> 20
   <212> ADN
   <213> Séquence artificielle
<220>
   <223> Oligonucléotide
<400> 3
   aaaggcctct aagacaagct 20
<210> 4
   <211> 20
   <212> ADN
   <213> Séquence artificielle
<220>
   <223> Oligonucléotide
<400> 4
   gccagcatgt gcaccgtgaa 20
<210> 5
   <211> 20
   <212> ADN
   <213> Séquence artificielle
<220>
   <223> Oligonucléotide
<400> 5
   ccgaagctta ctcacaattt 20